# EUROPÄISCHE PATENTSCHRIFT

(11) **EP 0 698 790 B1**
(45) Veröffentlichungstag und Bekanntmachung des Hinweises auf die Patenterteilung: **08.12.1999**
(21) Anmeldenummer: 94113419.9
(22) Anmeldetag: 27.08.1994
(51) Int. Cl.: G01N 33/00

(54) **Anordnung von Sensoren zur Messung der Luftfeuchte**
Disposition of sensors for humidity measurement
Dispositif de capteurs en vue de mesurer l'humidité atmosphérique

(43) Veröffentlichungstag der Anmeldung: 28.02.1996
(73) Patentinhaber: Centrum für intelligente Sensorik Erfurt e.V. ( CiS Erfurt e.V.), D-99097 Erfurt (DE)
(72) Erfinder: Heinze, Dirk, D-98693 Ilmenau (DE); Hoppe, Helmut, D-98693 Ilmenau (DE); Schmidt, Andreas, D-99089 Erfurt (DE); Steinke, Arndt, D-99192 Ingersleben (DE)
(74) Vertreter: Liedtke, Klaus, Dr.

(56) Entgegenhaltungen:
- EP-A- 0 325 050
- WO-A-94/10559
- DE-A- 3 519 436
- GB-A- 2 117 123
- GB-A- 2 203 249
- PATENT ABSTRACTS OF JAPAN vol. 9, no. 156 (P-368) (1879) 29. Juni 1985 & JP-A-60 031 049 (MATSUSHITA DENKO K. K.) 16. Februar 1985
- PATENT ABSTRACTS OF JAPAN vol. 7, no. 82 (P-189) (1227) 6. April 1983 & JP-A-58 010 642 (MURATA SEISAKUSHO) 21. Januar 1983

## Beschreibung

Die Erfindung betrifft eine Anordnung zur Messung der Gasfeuchte, der relativen Feuchte, der Taupunkttemperatur, der Betauung und der Luftzusammensetzung mit einer hohen Lebensdauer und im hohen Maße möglichen integrierten Form der Verarbeitung von Querempfindlichkeiten bestehend aus einem mikroelektronisch hergestellten Chip mit integrierten Sensoren.

Bekannter Stand der Technik sind eine ganze Anzahl von Meßverfahren zur Feuchtemessung speziell gasförmiger Medien.
Dabei sind die stationär betriebenen Meßverfahren wie Taupunktspiegel-Hygrometer, LiCl-Taupunkt-Hygrometer, bzw. kapazitive Taupunkt-Hygrometer auf der Basis der sich ändernden Materialeigenschaften (Dielektrizitätskonstante) bei Feuchtigkeitsaufnahme dominant. Diese dem Stand der Technik bekannten Anordnungen haben den Nachteil, daß die entsprechende dem Medium ausgesetzte Sensoranordnung bei Ausfall dieses einzigen verfügbaren Sensors gleichzeitig einen Ausfall des Sensormoduls nach sich zieht. Die Folge sind erforderliche Reparaturen bzw. der Austausch des Sensors.
Nach DE 37 20 189 A 1 wird als Sensor ein Streufeldkondensator zur Messung der Impedanz bzw. Kapazität beschrieben. Analog findet man bei DE 32 31 534 A 1 einen Streufeldkondensator, bei dem eine Elektrode gleichzeitig für die Temperaturmessung vorgesehen ist. Auch DE 34 46 277 A 1 sowie DE 34 16 945 A 1 enthalten Lösungen, bei denen jeweils ein Sensor zur Leitfähigkeitsmessung bzw. zur Temperaturmessung beschrieben wird.
Damit ist die Arbeitsmöglichkeit für sich ändernde Umgebungsbedingungen erheblich eingeschränkt. Ferner ist es bei Ausfall eines Sensors erforderlich, einen aufwendigen Austausch des Sensors vorzunehmen, was in der Regel auch mit einer Neukalibrierung verbunden ist.
Auch Feuchtemeßprinzipien, die das Taupunktspiegel-Verfahren nutzen, besitzen nur eine Sensoroberfläche, wobei sich hier derzeit eine Integration der Temperaturmessung der Spiegeloberfläche ausschließt.
Bei dem im Stand der Technik bekannten Anordnungen ist ein hoher Aufwand bei der Regelung, bei der Kalibrierung, bei der Berücksichtigung von Querempfindlichkeiten, bei der Darstellung thermodynamischer Zusammenhänge erforderlich.

Die bekannten Beispiele können nur singulär ein einziges der Feuchtemeßtechnik anhaftendes Problem lösen. Der Arbeitszeit- und Materialaufwand für derartige hybride Lösungen ist erheblich.

Nach WO-A-94 10559 ist eine Anordnung zur Wandlung nichtelektrischer Größen in elektrische Größen bekannt, die ein Erkennungssystem mit einem Gassensor beinhaltet, bei dem mehrere verschiedene selektive Schichten angebracht sind. Nachteilig ist hierbei, daß ein Aufheizen auf Temperaturen von ca. 450 °C erfolgt.

Ferner ist in JP-A-60031049 eine Anordnung von hybriden Sensoren beschrieben, bei der nach Ausfall eines Sensors auf einen andern Sensor umgeschaltet werden kann. Nachteilig ist hierbei, daß die Anordnung nicht in Mikrostrukturen ausgeführt werden und daß eine Heizung benötigt wird.

Alle im Stand der Technik bekannten Lösungen weisen die in der Feuchtemeßtechnik bedingten Anfälligkeiten der Sensoren bezüglich Ausfall durch Korrosion, Verschmutzungsempfindlichkeit und damit Drift des Arbeitspunktes sowie Unsicherheiten der Detektion von Wasser/Eis auf.

Der Erfindung liegt die Aufgabe zugrunde, die Möglichkeiten der hochintegrierten Signalverarbeitung mit einer die wichtigsten Querempfindlichkeiten ohne Meßwertverfälschung erfassenden Sensoranordnung zu verbinden.

Die Aufgabe wird erfindungsgemäß mit den kennzeichnenden Merkmalen von Patentanspruch 1 gelöst.

Vorteilhafte Ausgestaltungen sind in den Unteransprüchen angegeben.

Es handelt sich um eine Anordnung von Sensoren, die sich durch eine hohe Zuverlässigkeit bzgl. Lebensdauer und Meßgenauigkeit auszeichnen. Zu diesem Zweck werden auf einem Sensor-Chip neben einer integrierten Signalverarbeitung mehrere gleich- oder verschiedenartige Sensoren gleicher oder verschiedenartiger Wirkprinzipien angeordnet. Diese können zur Messung der Luftfeuchte für den stationären oder auch den statischen Betriebsfall geeignet sein.
Dabei werden unter Nutzung mikroelektronischer Schicht- und Strukturierungsverfahren sowie der Möglichkeit von integrierten Signalverarbeitungen eine hohe Langzeitstabilität und Zuverlässigkeit erreicht, wobei gleichzeitig die Vorraussetzungen gegeben sind, Querempfindlichkeiten zu berücksichtigen.

Ein wesentliches Ziel der Erfindung besteht in der Messung der Gasfeuchte. Bei der erfindungsgemäßen Anordnung werden die Mängel und Nachteile der dem Stand der Technik entsprechenden Lösungen vermieden.
Die Anordnung zeichnet sich aus durch:
- eine höhere Lebensdauer,
- eine integrierte Signalverarbeitung sowie integrierte Sensoren höherer Komplexität,
- Verarbeitungsmöglichkeit von Querempfindlichkeiten vor Ort,
- Verschmutzungsunempfindlichkeit,
- Erfassung von komplexen Meßgrößen sowie
- Möglichkeit der Unterscheidung von Wasser und Eis.

Bei dem erfindungsgemäßen Feuchtesensor befinden sich auf dem der Umwelt ausgesetzten Sensorfläche eine Anzahl von Sensoren, die zur Feuchtemessung unmittelbar verwendet werden können wie z.B.
- kapazitive Sensoren zur Messung der Änderung der Dielektrizitätskonstante bei Zunahme des Wasseranteils
- Impedanzsensoren zur Messung der Änderung des Widerstandes bei Zunahme des Wasseranteils
- Sensoren, die optimal für die Messung des Phasenwinkels dimensioniert worden sind als auch mittelbar für die Feuchtemessung notwendig sind wie z.B. integrierte pn-Übergänge oder Dünnschichtsysteme zur Messung der Temperatur bspw. als Taupunkttemperatur- oder als Umgebungstemperatursensor.

Mit der erfindungsgemäßen Anordnung wird erreicht, den allen Feuchtesensoren innewohnenden Nachteil der begrenzten Lebensdauer zu beseitigen bzw. erheblich einzuschränken.
Während bisher bei Ausfall eines Sensors ein Austausch vorgenommen werden mußte, entfällt diese zeitaufwendige Arbeit bei dieser erfindungsgemäßen Anordnung weitestgehend. Damit entfallen Zeiträume, in denen für Prozeßsteuerungen kein echtes Sensorsignal zur Verfügung steht. Auch Neukalibrierungen sind nach der hier beschriebenen Lösung nicht mehr erforderlich. Mittels der mikroelektronischen Schicht- und Strukturierungsverfahren ist es möglich, eine große Anzahl in ihren physikalischen, elektrischen oder mechanischen Eigenschaften identischer Sensoren unterschiedlicher Wirkprinzipien auf einem Sensorchip zu realisieren.
Diese Sensoren benötigen keine getrennte aufwendige Kalibrierung bzw. keinen Abgleich, da sie innerhalb des gleichen Prozesses hergestellt wurden und den Parameteranforderungen des integrierten Schaltkreises des Chips entsprechen. Darüber hinaus können die sensorspezifischen lithografischen Ebenen höheren Anforderungen (design rules) entsprechen, wenn mikroelektronisch kompatible Verfahren höherer Packungsdichte zur Anwendung kommen.
Diese Sensoren sind während des Herstellungsprozesses über die entsprechenden Schichtsysteme mit der integrierten Signalverarbeitung in einen elektrisch stabilen Kontakt gebracht worden, so daß sich eine spätere metallurgische Kontaktierung erübrigt.
Ist ein in Betrieb befindlicher Sensor von der integrierten Signalverarbeitung nicht mehr verwertbar, so erfolgt erfindungsgemäß eine chipinterne oder auch von außen anwählbare elektronische Kontaktierung auf den nächsten Sensor. Die mögliche Anzahl derartiger "Ersatzsensoren" richtet sich nach der zur Verfügung stehenden Chipfläche bzw. wird von der durch die minimale Strukturbreite möglichen Packungsdichte der Sensoren bestimmt.

Die Erfindung wird im Folgenden an einem Ausführungsbeispiel näher erläutert. In den zugehörigen Zeichnungen zeigen:
Figur 1: eine Draufsicht auf einen erfindungsgemäßen Sensorchip,
Figur 2.1: einen Schnitt durch einen Teil des Feuchtesensors,
Figur 2.2: einen Schnitt durch einen Teil des Feuchtesensors mit geänderten geometrischen Abmessungen auf dem gleichen Chip und die
Figuren 3.1 und 3.2: die Anordnungsmöglichkeiten der Sensoren neben bzw. über der integrierten Signalverarbeitung.

Die erfindungsgemäße Anordnung stellt die integrierten und in Dünnschichttechnologien hergestellten Sensoren neben der integrierten Signalverarbeitung dar, die gemeinsam Bestandteil eines Silizium-Chips sind.
Die erfindungsgemäßen Sensoranordnungen 4 weisen einen Träger 1 aus Silizium auf, der aus dem gleichen Grundmaterial, wie der Teil des integrierten Schaltkreises besteht, vorzugsweise aus p- oder n- leitendem einkristallinen Silizium.
Auf diesem Grundträger befindet sich eine Isolatorschicht 2 mit ausreichender Dicke. Die Isolatorschicht 2 ist vorzugsweise thermisch gewachsenes Siliziumdioxid, das dadurch durch Hochtemperatur-CVD- oder Niedertemperatur-CVD-Isolatorschichten aus Siliziumdioxid, Siliziumnitrid oder Oxynitriden verstärkt wurde. Dabei spielen diese Schichten eine besondere Rolle in der Form, daß sie die Spannung aus dem Silizium bzw. von der Sensoroberfläche aufnehmen und ausgleichen müssen. Selbst müssen diese Schichten feuchtigkeitsunempfindlich sein und auch keine chemischen Substanzen wie beispielsweise Phosphor enthalten, die bei Eindringen von Feuchtigkeit eine erhöhte chemische Aggressivität mit sich bringen. Bis auf in Silizium integrierte Sensoren, wie beispielsweise pn-Di-oden, sollten in diesem Bereich keine metallurgische Verbindungen, wie beispielsweise Kontakte zwischen mehreren leitfähigen Schichten und Bereichen, vorliegt.

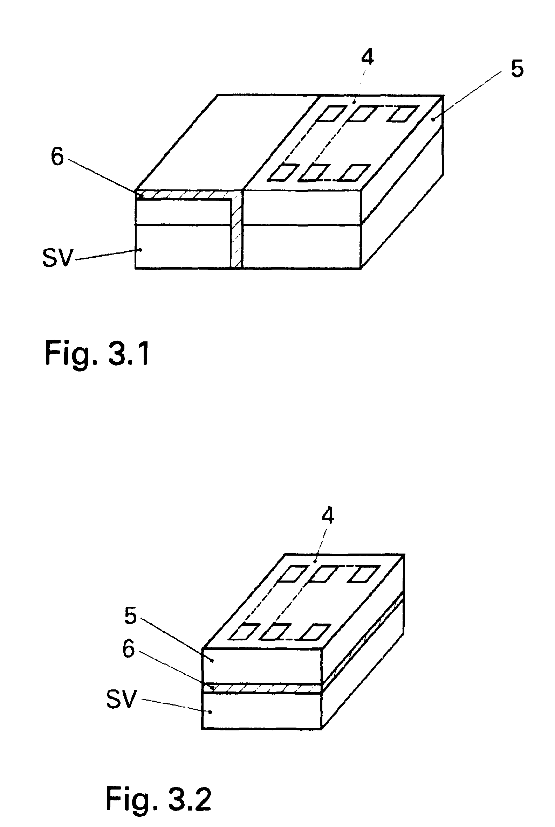

Auf der Sensoroberfläche 4 sind mehrere für sich getrennte, funktionsfähige Sensoren angeordnet. Die Sensoren S 11 ... S 1n, S 21 ... S 2m,...., Sx1....Sxy verkörpern jeweils Sensoren mit gleichen elektrischen, chemischen bzw. physikalischen Wirkprinzip. Sie können sich in ihrer geometrischen Dimensionierung (S11... S1n, S21...S2m,...., Sx1...Sxy) unterscheiden. Für eine bevorzugte Ausführungsform ist die Dimensionierung der S11...S1n, S21...S2m,...,Sx1...Sxy- Sensoren jeweils identisch, so daß eine nahezu 100%ige Kompatibilität und Austauschbarkeit für die integrierten Signalverarbeitung besteht. Die einzelnen Sensoren werden vorzugsweise im Bereich der integrierten Signalverarbeitung an die integrierte Schaltung kontaktiert.
Die integrierte Schaltung beinhaltet auf Grund der flächenhaften Anordnung zur Sensoroberfläche gemäß Figur 3.1 vorzugsweise eine Signalvorverarbeitung, beispielsweise Operationsverstärker, Multiplexer, Komperatoren, oder Kapazität-Frequenz-Wandler. Diese niedrig integrierte Form ist in diesem Fall zu wählen, da bei der relativ großen Chipfläche der Einfangquerschnitt für Defekte groß ist und die Wahrscheinlichkeit, ein funktionsfähiges Chip auf dem Wafer vorzufinden, gering ist. In der Ausführungsform gemäß Figur 3.2, in der die integrierte Signalverarbeitung unterhalb der Sensorfläche angeordnet ist, bestehen andere Bedingungen. Hierbei wurde eine kundenspezifische Schaltung in das Silizium mit einer hohen Funktionsausbeute gebracht. Sie enthält über der aktiven Fläche die eigentlichen Sensoren. Zwischen den Sensoren und der Signalverarbeitung wird eine optimale elektrische, chemische, physikalische und mechanische Barrierenschichten realisiert. Diese Anordnung gestattet die Integration von digitalen und analogen Schaltungen höchsten Integrationsgrades und optimaler Schnittstellen, beispielsweise zu peripheren Datennetzen und Aktoren. Darüber hinaus ist chipintern eine intelligente Signalverarbeitung möglich, die
- die Verarbeitung von Querempfindlichkeiten vornimmt,
- die Drift des Arbeitspunktes berücksichtigt,
- den Ausfall von Sensoren erkennt und zeitecht reagiert,
- nichtlineares Verhalten bestimmter Parameter mit abgelegten Kennlinien vergleicht und verarbeitet sowie
- Veränderungen von physikalischen Eigenschaften des Wassers durch Phasenübergang flüssig - fest erkennt.

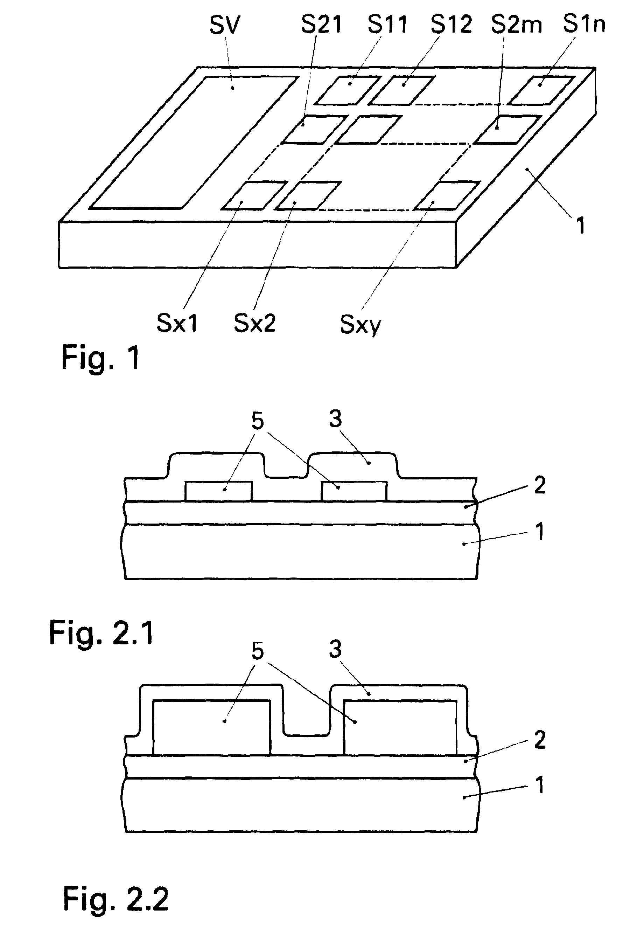

Wie aus Figur 1 ersichtlich ist, sind die Sensoren (n) z.B. Interdigitalstrukturen, die als Streufeldkondensator mit den Elektroden 5 wirken, wenn sie mit einer feuchtigkeitsunempfindlichen Schicht 3 überzogen sind. Bei Betauung der Sensoroberfläche ändert sich dann auf Grund der Änderung der Dielektrizitätskonstante die Gesamtkapazität. Auch lassen sich bei dieser Änderung über die Bestimmung des Phasenwinkels grobe Angaben über Verschmutzungen der Sensoroberfläche machen. Unterschiedlich dimensionierte Streufeldkondensatoren auf dem gleichen Chip, wie in den Figuren 2.1 und 2.2 angegeben, erlauben unterschiedliche Empfindlichkeiten bezüglich des kapazitiven oder des Impedenzanteils in der integrierten Signalverarbeitung zu detektieren.

Die m-Sensoren könnten zur Leitfähigkeitsbestimmung herangezogen werden. Insbesondere zur Wasser/Eis-Unterscheidungen sind diese Sensoren günstig.
Weitere sensorische Wirkprinzipien könnten integrierte oder Dünnschichttemperatursensoren, Magnetfeld- oder auch Lichtsensoren darstellen. Verbunden mit dem Einsatz von sorptiven Materialien ist die Integration von Sensoren zur Messung der relativen Feuchte ebenfalls möglich.

### BEZUGSZEICHENLISTE

- 1: Silizium-Träger
- 2: Isolatorschicht
- 3: Feuchteresistente Schutzschicht
- 4: Sensor-Fläche
- 5: Sensor-Elektrode
- 6: Barrieren-Schicht
- SV: Integrierte Signalverarbeitung
- S: Integrierte Sensoren

## Patentansprüche

1. Anordnung zur Messung der Gasfeuchte, der relativen Feuchte, der Taupunkttemperatur, der Betauung und der Luftzusammensetzung mit einer hohen Lebensdauer und im hohen Maße möglichen integrierten Form der Verarbeitung von Querempfindlichkeiten bestehend aus einem mikroelektronisch hergestellten Chip mit integrierten Sensoren, **dadurch gekennzeichnet,**
- daß die für die Sensoren vorgesehene Fläche aus einer Vielzahl gleichartig dimensionierter Sensoren besteht, die in einer Interdigitalstruktur angeordnet sind,
- daß die Sensoren mit einer integrierten Signalverarbeitung verbunden sind und
- daß Mittel vorgesehen sind, mit denen bei Ausfall eines der gleichartigen Sensoren auf einen anderen gleichartigen Sensor schaltungsseitig übergegangen wird.

2. Anordnung nach Anspruch 1, **dadurch gekennzeichnet,** daß Mittel vorgesehen sind, mit denen einzelne Sensoren unterschiedlicher Wirkprinzipien, die sich auf der gleichen Sensorfläche befinden, zugeschaltet werden.

3. Anordnung nach Anspruch 1 oder 2, **dadurch gekennzeichnet,** daß die integrierte Signalverarbeitung flächenhaft neben der Sensorfläche angeordnet ist.

4. Anordnung nach Anspruch 1 oder 2, **dadurch gekennzeichnet,** daß die integrierte Signalverarbeitung unterhalb der Sensorfläche angeordnet ist.

5. Anordnung nach einem der vorhergehenden Ansprüche, **dadurch gekennzeichnet,** daß die Sensoren gleichen Wirkprinzips verschiedenartig dimensioniert sind.

6. Anordnung nach einem der vorhergehenden Ansprüche, **dadurch gekennzeichnet,** daß für die Wasser/Eis-Detektion bei Taupunktsensoren neben auf der Basis von Streufeldkapazitäten arbeitenden Sensoren auch weitere Sensoren, die sich auf der gleichen Sensorfläche befinden, angeordnet sind.

## Claims

1. Arrangement for measuring gas humidity, relative humidity, temperature of dew point, dewing and air composition with a long service life and allowing a high degree of integrated processing of cross-axis sensitivity information, consisting of a chip made by microelectronic manufacturing with integrated sensors, **characterized in that**
- the surface provided for the sensors consists of a multitude of sensors dimensioned in the same way and arranged in an interdigital structure;
- that the sensors are connected to an integrated signal processing and in that
- means are provided by means of which the arrangement is shifted to another sensor of the same kind on the switching side in case of failure of one of the sensors of the same kind.

2. Arrangement as claimed in claim 1, **characterized in that** means are provided by means of which individual sensors of differing operating principle located on the same sensor surface are switched on additionally.

3. Arrangement as claimed in claim 1 or 2, **characterized in that** the integrated signal processing is located in a planar form next to the sensor surface.

4. Arrangement as claimed in claim 1 or 2, **characterized in that** the integrated signal processing is located below the sensor surface.

5. Arrangement as claimed in any of the above-mentioned claims, **characterized in that** the sensors of the same operating principle are dimensioned differently.

6. Arrangement as claimed in any of the above-mentioned claims, **characterized in that** additional sensors which are located on the same sensor surface are arranged in addition to sensors working on the basis of stray-field capacities for water/ice detection in dew-point sensors.

## Revendications

1. Dispositif de mesure de l'humidité gazeuse, de l'humidité relative, de la température du point de rosée, de la rosée et de la composition de l'air, à longue durée de vie et comportant dans une grande mesure une forme intégrée possible de traitement de sensibilités transversales, consistant en une puce micro-électronique fabriquée à senseurs intégrés, **caractérisé en ce que**
- la surface de senseurs prévue consiste en plusieurs senseurs dimensionnés identiquement, lesquels sont disposés dans une structure interdigitale,
- les senseurs sont reliés à un traitement de signal intégré, et en ce que
- des moyens sont prévus, en cas de panne de l'un des senseurs identiques, pour une commutation de circuit sur un autre senseur identique.

2. Dispositif selon revendication 1, **caractérisé en ce que** des moyens sont prévus, par lesquels des senseurs isolés à principes d'action différents se trouvant sur la même surface de senseurs sont mis en circuit.

3. Dispositif selon revendication 1 ou 2, **caractérisé en ce que** le traitement de signal intégré est placé en surface à côté de la surface de senseurs.

4. Dispositif selon revendication 1 ou 2, **caractérisé en ce que** le traitement de signal intégré est placé sous la surface de senseurs.

5. Dispositif selon l'une des revendications précédentes, **caractérisé en ce que** les senseurs à même principe d'action sont différemment dimensionnés.

6. Dispositif selon l'une des revendications précédentes, **caractérisé en ce que** pour la détection d'eau/de glace sont présents, dans le cas de senseurs à point de rosée, outre des senseurs fonctionnant sur le principe du champ de dispersion, d'autres senseurs disposés sur la même surface de senseurs.
